# EUROPEAN PATENT SPECIFICATION

(11) **EP 3 681 580 B1**
(45) Date of publication and mention of the grant of the patent: **14.05.2025**
(21) Application number: 18854017.3
(22) Date of filing: 11.09.2018
(51) Int. Cl.: A61M 25/092, A61M 25/01, A61M 25/14

(54) **CATHETER DEVICE FOR LUMEN RE-ENTRY**
KATHETERVORRICHTUNG FÜR LUMENWIEDEREINTRITT
DISPOSITIF DE CATHÉTER POUR RÉ-ENTRÉE DE LUMIÈRE

(30) Priority: 11.09.2017 US 201762556610 P
(43) Date of publication of application: 22.07.2020
(73) Proprietor: Sunnybrook Research Institute, Toronto, Ontario M4N 3M5 (CA)
(72) Inventor: TENNANT, Ryan, Kitchener Ontario N2A 3C2 (CA); DI BARTOLOMEO, Lindsey, Concord Ontario L4K 5S4 (CA); STRAUSS, Bradley, Toronto Ontario M6B 1T4 (CA); ABUZEID, Wael, Toronto Ontario M4S 3H8 (CA); ARDESHIRI, Ramtin, Richmond Hill Ontario L4E 4C4 (CA); CIBULSKI, Gilad, 6092000 Kadima (IL)
(74) Representative: Hertin und Partner Rechts- und Patentanwälte PartG mbB
(86) International application number: PCT/CA2018/051124
(87) International publication number: WO 2019/046976

(56) References cited:
- WO-A2-2011/025855
- US-A1- 2004 039 371
- US-A1- 2012 265 233
- US-A1- 2013 006 167
- US-A1- 2013 072 957
- US-B2- 8 608 688
- US-B2- 8 617 231
- US-B2- 9 277 923
- US-B2- 9 402 981

## Description

This application claims the benefit of U.S. Provisional Patent Application Serial No. 62/556,610, filed on September 11, 2017, and entitled "CATHETER DEVICE CATHETER DEVICE FOR LUMEN RE-ENTRY AND METHODS FOR USE THEREOF".

### BACKGROUND

Chronic total occlusions (CTO] can be found in coronary angiography, occurring in approximately 18-33% of patients with documented coronary artery disease. Percutaneous revascularization (angioplasty] is attempted in less than 10% of CTOs. Approximately 25% of cases undergo bypass surgery, while the majority (approximately 65%] are treated medically. The main reasons for not attempting percutaneous revascularization include the frequent presence of multi-vessel disease, and the complexity and time requirements of performing these technically challenging percutaneous procedures.

About 70% of percutaneous revascularization procedures are successful.
This is primarily due to the difficulty in crossing the occlusion with guidewires in the antegrade direction. A challenge is crossing the fibrotic and often calcified material that is occluding the artery and then re-entering the true lumen beyond the occluded segment. In some cases, the guidewire immediately re-enters the true lumen at the end of the CTO (just past the distal end], which is known as true-true crossing. However, in many cases, the guidewire cannot cross the occlusion, but is in a subintimal position after the occlusion and has to re-enter the true lumen further downstream - so called true to false to true. In some cases, downstream re-entry can be done by reshaping the tip of CTO specialty guidewires advanced through a central lumen microcatheter (such as finecross or corsair] and directing the guidewire back into the true lumen. Also, angulated microcatheters can be used, but control of the angle of the tip can be challenging in the subintimal space.

More recently, a specialized CTO device has been introduced that first involves advancing a catheter (known as the CrossBoss], which is a proximal torque device that utilizes bidirectional rotation with a fast-spin technique, in order to advance across the occlusion as the spin reduces the push required. Although this catheter can be advanced within the luminal space, it is usually advanced within the subintimal space and then the CrossBoss catheter is replaced with a special flat balloon that has two holes at different orientations (Stingray balloon] through which the operator advances a very stiff guidewire (Stingray wire] to re-enter the artery. This is a technically challenging procedure that requires extensive training and has been restricted so far to highly expert operators.

Subintimal positioning of the guidewire (i.e. inside the wall of the coronary artery rather than the true lumen] after crossing the occlusion is a major problem and common failure more in CTO PCI, and highlights the need for additional options to facilitate re-entry into the true lumen of the artery after the occlusion.
US patent application No. 2013/072957 A1 discloses a catheter for recanalizing a blood vessel having an occlusion therein via a subintimal pathway. The catheter includes a catheter shaft having an inflatable balloon mounted to the distal end portion of the catheter shaft. A flexible tubular member extends from the catheter shaft and along an exterior of the inflatable balloon Inflation of the inflatable halloon deflects the flexible tubular member into a deflected configuration away from a longitudinal axis of the catheter shaft to effect re-entry into the true lumen distal of the occlusion.
International patent application publication No. WO2011/025855A2 discloses a method for thermal bonding of an inverted balloon neck on a catheter, including placing an inverted balloon neck on a shaft of a catheter, and characterized by applying heat at an internal hollow of the shaft where the inverted balloon neck is placed, while applying internal pressure to attach an external surface of the shaft (86) to the inverted balloon neck.
US patent application No. 2013/006167A1 discloses a method and apparatus for crossing an obstruction in a tubular member, and more particularly to a medical device method for crossing of a chronic occlusion in a subintimal or interstitial space of an artery.

### SUMMARY OF THE INVENTION

The present invention is defined in independent claim 1 and subsequent dependent claims 2 - 15.

### SUMMARY OF THE DISCLOSURE

The present disclosure addresses the aforementioned challenges by providing a catheter device designed for lumen re-entry, which may be used for percutaneous CTO revascularization and other applications, such as angioplasty procedures where it can be challenging to position a guidewire in a side branch vessel with a difficult angulation.

It is an aspect of the present disclosure to provide a catheter that includes a catheter shaft having a tubular wall that extends from a proximal end to a distal end along a longitudinal axis to define a lumen. The tubular wall having formed therein an inner lumen that extends from the proximal end to the distal end of the catheter shaft. The catheter device also includes a deflection member coupled to the distal end of the catheter shaft and in fluid communication with the inner lumen such that fluid provided to the inner lumen causes the deflection member to expand from a first volume to a second volume that is larger than the first volume. When the deflection member is in the second volume, it extends from a surface of the tubular wall towards the longitudinal axis of the catheter shaft to provide a surface for deflecting an interventional device extending through the lumen and outward from the distal end of the catheter shaft at a deflection angle.

The foregoing and other aspects and advantages of the disclosure will appear from the following description. In the description, reference is made to the accompanying drawings which form a part hereof, and in which there is shown by way of illustration a preferred embodiment of the disclosure. Such embodiment does not necessarily represent the full scope of the disclosure, however.

### BRIEF DESCRIPTION OF THE DRAWINGS

FIG. 1A is a cross sectional view of an example of a distal portion of a catheter device having an expandable member;
FIG. IB is a cross sectional view of the example catheter device of FIG. 1A in which a wire is positioned therethrough;
FIGS. 2A-2D show example configurations of deflection members that can be used with the catheter device described in the present disclosure.
FIG. 3 is an axial cross-sectional view of an example of a distal portion of the catheter device of FIG. 1A having an expandable member at a first volume;
FIG. 4 is an axial cross-sectional view of an example of a distal portion of the catheter device of FIG. 1A having an expandable member at a second volume;
FIG. 5 is a cross-sectional view of another example of a distal portion of a catheter device having an expandable member;
FIG. 6 is a cross sectional view of the example catheter device of FIG. 3 in which a wire is positioned therethrough;
FIG. 7 is a perspective view of the example catheter device of FIG. 3
FIG. 8 is a cross-sectional view of another example of a distal portion of a catheter device having an expandable member;
FIG. 9 is a perspective view of an example marked portion of any of the FIGS. 1 to 8 positioned in which a "C" shape is visible;
FIG. 10 is an example of a medical image in which the "C" shape of FIG. 9 is visible;
FIG. 11 is a perspective view of an example marked portion of any of the

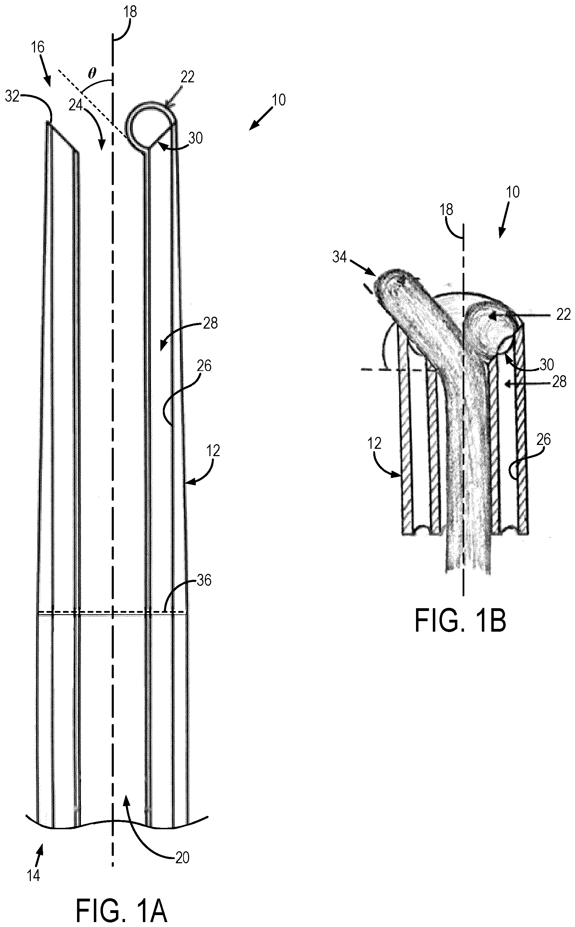

   FIGS. 1 to 8 positioned in which a "Z" shape is visible;

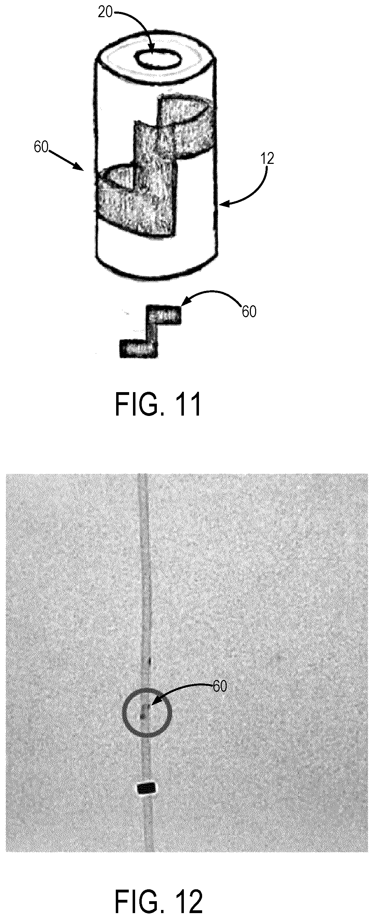

FIG. 12 is an example of a medical image in which the "Z" shape of FIG. 11 is visible;

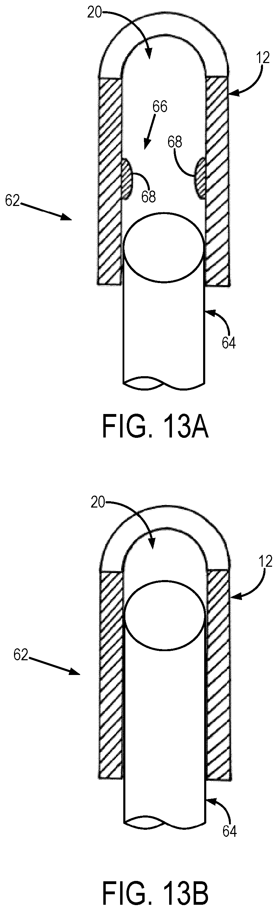

FIG. 13A is a cross-sectional view of an example of a catheter device configured to interface with a catheter realignment system;

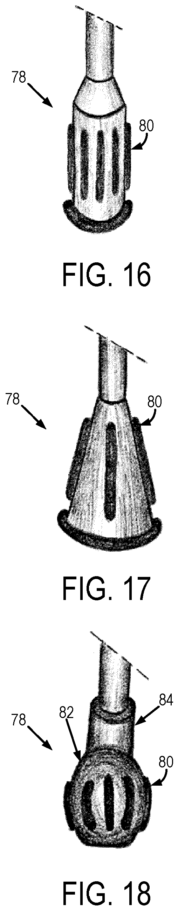

FIG. 13B is a cross-sectional view of the example catheter device of FIG. 16A having a rod fed therethrough;

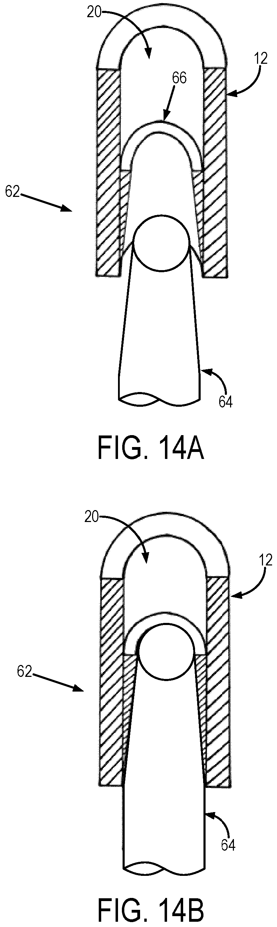

FIG. 14A is a cross-sectional view of an example of a catheter device configured to interface with a catheter realignment system;
FIG. 14B is a cross-sectional view of the example catheter device of FIG. 17A having a rod fed therethrough;

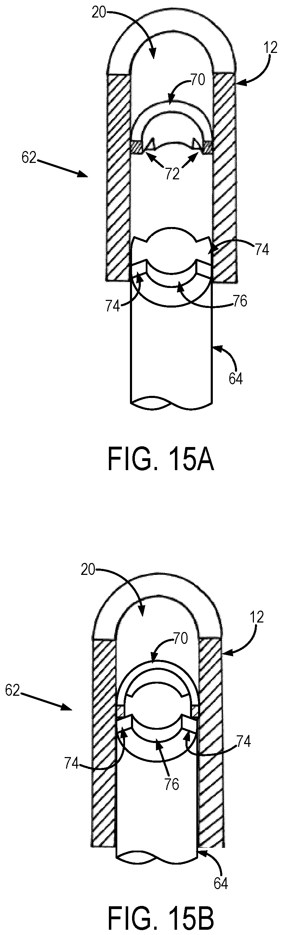

FIG. 15A is a cross-sectional view of an example of a catheter device configured to interface with a catheter realignment system;
FIG. 15B is a cross-sectional view of the example catheter device of FIG. 18A having a rod fed therethrough;
FIG. 16 is a perspective view of an example of a proximal end of a catheter realignment mechanism having a handle and a rod;
FIG. 17 is a perspective view of another example of a proximal end of a catheter realignment mechanism having a handle and a rod;
FIG. 18 is a perspective view of another example of a proximal end of a catheter realignment mechanism having a handle and a rod;

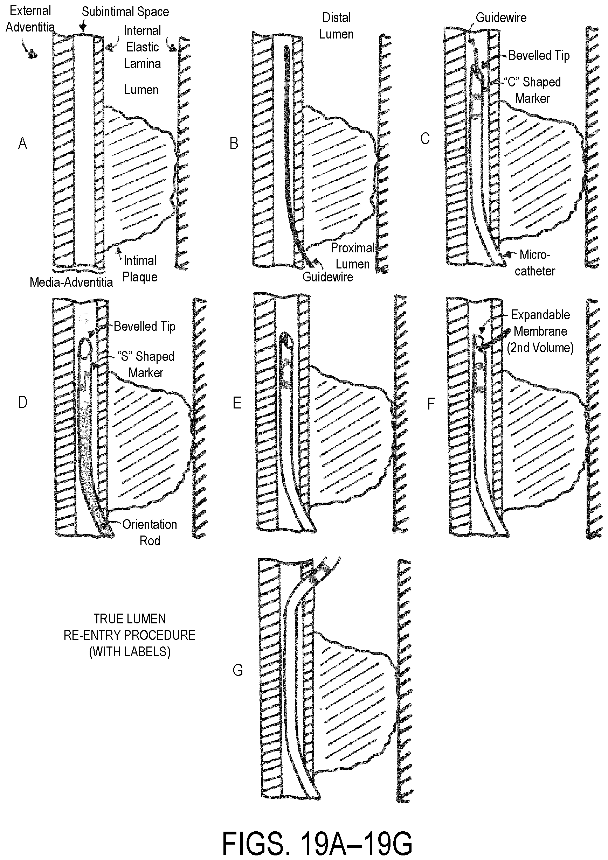

FIGS. 19A-19G show the steps of an example subintimal re-entry procedure using an example of a catheter described in the present disclosure; and

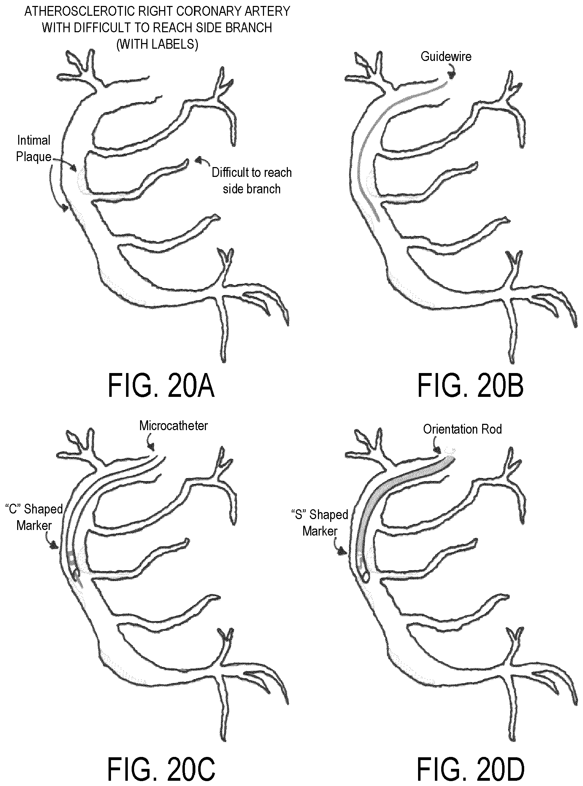

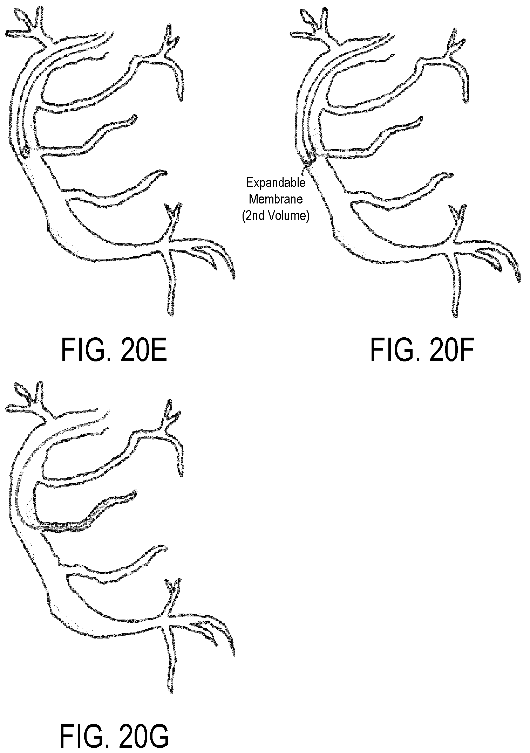

FIGS. 20A-20G show the steps of an example procedure for accessing a difficult side-branch using an example of a catheter described in the present disclosure.

### DETAILED DESCRIPTION

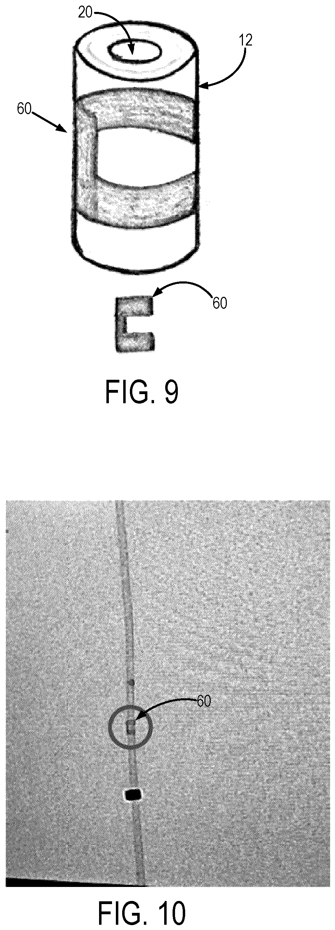

By way of overview and introduction, a catheter device that can provide subintimal orientation and re-entry into a lumen is generally illustrated in FIGS. 1-18. As will be described, one advantageous clinical use of the catheter device is treatment of chronic total occlusions ("CTO"]. The catheter device can also be used for other vascular treatment applications, such as the placement of stents and angioplasty balloons, deflection of guidewires into angulated side branches from an intraluminal position, orientation of a guidewire at the beginning of occlusion to engage a CTO proximal to the CTO and at an angle that is not parallel to the artery, and non-vascular treatment applications. Generally, the catheter device can include an orientation subsystem and a re-entry subsystem. Embodiments of the re-entry subsystem will be described with respect to FIGS. 1-8. Embodiments of the orientation subsystem will be described with respect to FIGS. 9-18.

Referring now to FIGS. 1A and 1B, the catheter 10 includes a catheter shaft
12 extending from a proximal end 14 to a distal end 16 along a longitudinal axis 18 to define a lumen 20. A deflection member 22 is coupled to the shaft 12 at the distal end 16 of the catheter 10. The deflection member 22 generally includes an expandable membrane that when filled with a fluid expands from a first volume (e.g., a deflated volume] to a second volume (e.g., an inflated or expanded volume]. When expanded to the second volume, the deflection member 22 provides a surface for deflecting a guidewire, or other interventional device, extending through the lumen 20 outward through a distal opening 24 of the catheter shaft 12. As will be described below, the expanded volume of the deflection member 22 provides a surface that will deflect the guidewire, or other interventional device, a deflection angle, θ , when exiting the opening 24 at the distal end 16 of the catheter 10.

The wall of the catheter shaft 12 has formed therein an inner lumen 26 extending from the proximal end 14 to the distal end 16 of the catheter 10. The inner lumen 26 receives a hypotube 28 that is in fluid communication with the deflection member 22. The deflection member 22 generally spans an aperture 30 formed as the distal end of the inner lumen 26, or any hypotube 28 provided to the inner lumen 26. The aperture 30 can be formed in the distal end of the inner lumen 26, as shown in FIGS. 1A and IB, or alternatively can be formed along the outer surface of the inner lumen 26 and through the catheter shaft 12 such that the deflection member 12 can be operated in a side-firing arrangement into the interior lumen 20 of the catheter shaft 12. Providing a fluid to the hypotube 28 causes the deflection member 22 to expand from the first volume to the second volume. By controlling the amount of fluid provided to the deflection member 22 via the hypotube 28, the second volume, and thereby the deflection angle, Θ , provided by the deflection member 22, can be similarly controlled. Thus, by controlling the expanded volume of the deflection member 22, a guidewire, or other interventional device, can be deflected into the true lumen of a blood vessel when the catheter 10 is positioned in the subintimal space.

In some other embodiments, more than one deflection member 22 can be provided at the distal end 16 of the catheter 10. In these configurations, a similar number of inner lumens 26 are formed in the wall of the catheter shaft 12 such that a different hypotube 28 may be provided to each inner lumen 26 to be in fluid communication with one of the deflection members 22.

In some embodiments, the wall of the catheter shaft 12 does not include an inner lumen 26; rather, the hypotube 28 is provided to the interior surface of the lumen 20 of the catheter shaft 12. In such configurations, it will be appreciated that more than one hypotube 28 can be similarly provided to the interior surface of the lumen 20 of the catheter shaft 12. It will also be appreciated that in some embodiments a hypotube 28 can be provided to both an inner lumen 26 formed in the wall of the catheter shaft 12, and to the interior surface of the lumen 20 of the catheter shaft 12 itself.

The wall of the catheter shaft 12 can be chamfered or beveled such that the wall slopes proximally toward the distal opening 24 of the catheter 10, thereby defining an angled surface 32. The angled surface 32, in turn, defines a maximum deflection angle at which a guidewire, or other interventional device, can be deflected upon exiting the opening 24 at the distal end 16 of the catheter 10. In some configurations, the exterior surface of the catheter shaft 12 may be inwardly tapered distal to a taper line 36 towards the distal end 16 of the catheter 10.

As mentioned above, and as shown in FIGS. 1A and IB, the catheter 10 can receive a guidewire 34 that can be fed through the lumen 20 from the proximal end 14 to the distal end 16 of the catheter 10. The guidewire 34 can be directed through the opening 24 at the distal end 16 of the catheter 10 via contact with the deflection member 22 and the angled surface 32 of the catheter shaft 12. That is, the guidewire 34 is deflected by contact with the deflection member 22 so as to extend outward from the distal opening 24 at the deflection angle.

The deflection member 22 is generally constructed as an expandable membrane that spans the aperture 30 in the catheter shaft 12 formed by the inner lumen 26. In some other configurations, the deflection member 22 can be constructed as an expandable membrane that spans the opening of the hypotube 28. That is, the deflection member 22 can be coupled to the catheter shaft 12 or to the hypotube 28. As described above, fluid is provided to the deflection member 22 via the hypotube 28 to expand the deflection member from a first volume to a second volume. The deflection member 22 can have a partially spherical shape; although, other shapes can also be implemented, such as partially ellipsoidal shapes and the like. The deflection member 22 can extend outwardly from the distal end 16 of the catheter 10 along a direction perpendicular, or nearly perpendicular, to the angled surface 32 of the catheter shaft 12.

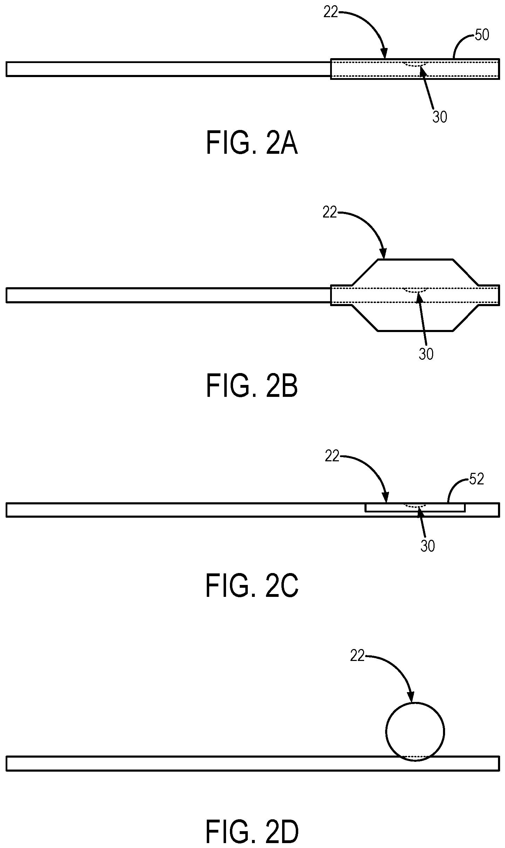

In some embodiments, the deflection member 22 can be constructed as an extruded sleeve that spans the aperture 30 in the catheter shaft 12 formed by the inner lumen 26. Alternatively, the extruded sleeve can span a first aperture that is formed in the hypotube 28, which is aligned with a suitable second aperture in the catheter shaft 12, such as aperture 30. An example of this configuration in an undeployed state is shown in FIG. 2A and in a deployed state in FIG. 2B. In this example, the aperture 30 in the catheter shaft 12 formed by the inner lumen 26 terminates on the inner surface of the catheter shaft 12 such that the deflection member 22 will be deployed in a side-firing configuration. The deflection member 22 is also shown in this example as a balloon that is inflated when fluid is provided through the hypotube 28 to the interior volume of the deflection member 22 in its undeployed state. As noted, the deflection member 22 in this example can be constructed as an extruded sleeve 50. The deflection member 22 can also be constructed by dipping the catheter shaft 12 or hypotube 28 in a suitable material to form the deflection member 22 in its undeployed state. The deflection member 22 can be composed of silicon, polyurethane, silicone polypropylene , PEBAX^{®} (Arkema; Colombes, France], or other suitable expandable material, which may include a polymer, elastomer, or so on.

In some other embodiments, the deflection member 22 can be constructed as a patch 52 made by an extruded sleeve that spans the aperture 30 in the catheter shaft 12 formed by the inner lumen 26. Alternatively, the patch 52 can span a first aperture that is formed in the hypotube 28, which is aligned with a suitable second aperture in the catheter shaft 12, such as aperture 30. The patch 52 can also be formed by dipping the catheter shaft 12 or hypotube 28 in an expandable material that spans the aperture 30 in the catheter shaft, or an aperture formed in the hypotube 28. An example of this configuration is shown in an undeployed state in FIG. 2C and in a deployed state in FIG. 2D. In this example, the aperture 30 in the catheter shaft 12 formed by the inner lumen 26 terminates on the inner surface of the catheter shaft 12 such that the deflection member 22 will be deployed in a side-firing configuration. The patch 52 can span a portion of the circumference of the catheter shaft 12 or hypotube 28, as shown in FIG. 21C. The deflection member 22 is also shown in this example as a balloon that is inflated when fluid is provided through the hypotube 28 to the interior volume of the deflection member 22 in its undeployed state. As noted, the deflection member 22 in this example can be constructed as a patch 52 made by an extruded sleeve or by dipping the catheter shaft 12 or hypotube 28 in a suitable material to form the deflection member 22 in its undeployed state. The deflection member 22 can be composed of silicon, polyurethane, silicone polypropylene, PEBAX^{®} (Arkema; Colombes, France], or other suitable expandable material, which may include a polymer, elastomer, or so on.

As described above, when a fluid is provided to the deflection member 22 via the hypotube 28, the deflection member 22 expands from the first volume to a second volume. When the deflection member 22 has the second volume it partially extends into the opening 24 at the distal end 16 of the catheter 10, thereby providing a surface that will deflect a guidewire, or other interventional device, passing through the opening 24 at the distal end 16 of the catheter 10. As one non-limiting example, when the deflection member 22 has the first volume, the deflection member 22 has a substantially flat shape. That is, the deflection member 22 can be relatively flat against the angled surface 32 of the catheter shaft 12. In some other embodiments, the deflection member 22 can be partially protruding from, or partially recessed relative to, the angled surface 32 of the catheter shaft 12. As the pressure supplied to the deflection member 22 by the fluid is decreased, the deflection member 22 can begin to compress and transition from the expanded shape at the second volume to the flatter shape against the angled surface 32 at the first volume. It should be appreciated that the deflection member 22 can be partially expanded, thereby expanding the deflection member 22 into an intermediate position.

In some embodiments, the catheter 10 is constructed to be a microcatheter.
As one example, the catheter 10 can be constructed as a microcatheter for use in coronary arteries. Thus, in some non-limiting examples, the catheter 10 can be sized at 4.5 Fr (1.5 mm] or less. As another example, the catheter 10 can be constructed to be a microcatheter for use in peripheral arteries, which can allow a larger outer diameter than in coronary microcatheter implementations.

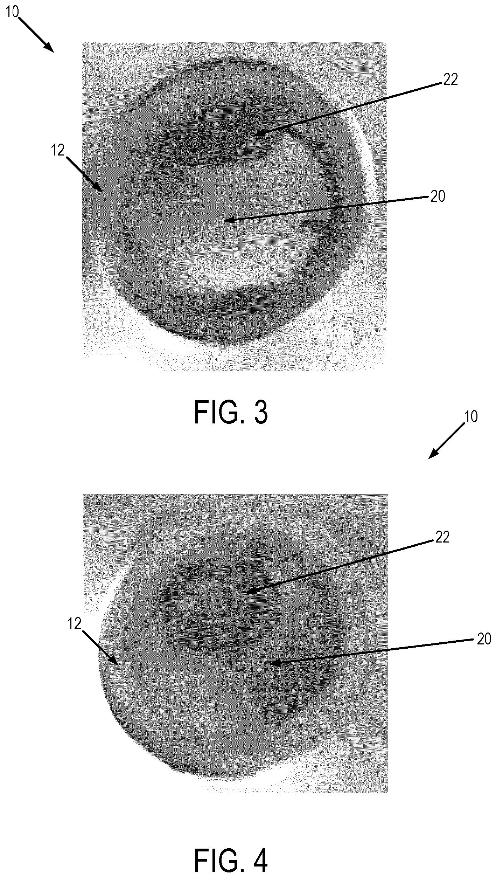

Referring to FIGS. 3 and 4, a view looking down the catheter shaft 12 from the distal end 16 of the catheter 10 is shown with the deflection member 22 at the first volume (FIG. 3] and at the second volume (FIG. 4]. As shown, the catheter shaft 12 can be a tubular structure that defines the lumen 20 within the tubular structure of the catheter shaft 12. The catheter shaft 12 is generally composed of a medical device class VI approved polymer material, such as, for example, polyethylene terephthalate ("PET"]; however, other polymer materials could also be employed, such as other related PET formulations, polyethylene naphthalate ("PEN"], polyether ether ketone ("PEEK"], and polyether block amide ("PEBA"], such as PEBAX^{®} (Arkema; Colombes, France].

In some embodiments, the catheter can be composed of more than one material. As one example, the catheter shaft can have first layer composed of a first material and a second layer composed of a second material. In such embodiments, the first layer can correspond to the inner surface of the catheter shaft 12 and the second layer can correspond to the outer surface of the catheter shaft 12. The first layer can be thin, such as 0.001", and the second layer can be molded around the first layer and any hypotubes 28 positioned in the wall of the catheter shaft 12. As one benefit, this two- layered composition can facilitate creating a varied outer diameter for the catheter shaft 12, such as creating a tapered outer surface for the catheter shaft 12, as described above. In these embodiments, the deflection member 22 can be molded into the first layer. In other embodiments, the deflection member 22 can be formed by applying a thin membrane (e.g., a thin membrane of latex plastic or other such material] across a hypotube 28.

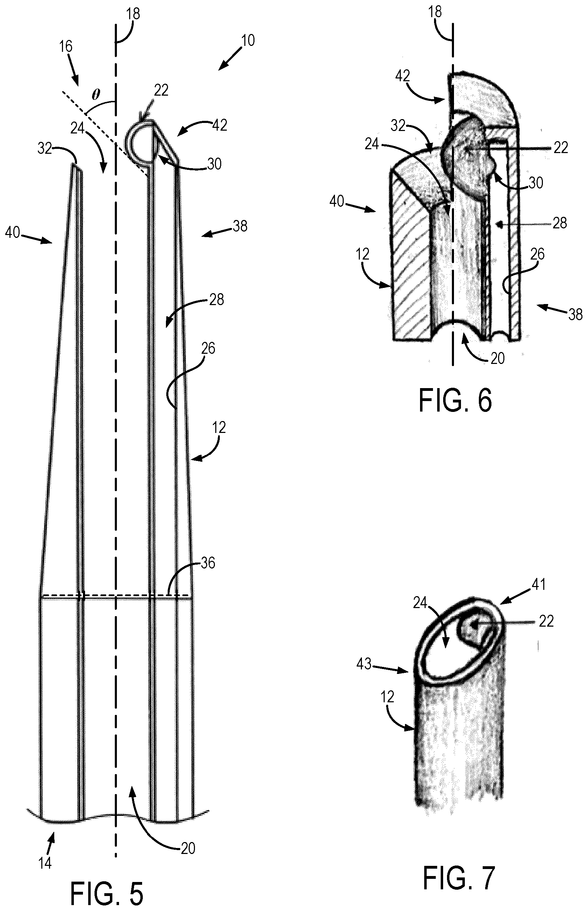

Referring to FIGS. 5 and 6, another example of a catheter 10 of the present disclosure is illustrated. In this example, the catheter 10 generally includes a catheter shaft 12 extending from a proximal end 14 to a distal end 16 along a longitudinal axis 18 to define a lumen 20. In this example, the catheter 10 is generally constructed such that the catheter shaft 12 includes a single inner lumen 26. As shown, the inner lumen 26 is generally positioned on a first side 38 of the catheter shaft 12. In some configurations, the second side 40 of the catheter shaft 12 can have a thinner outer wall than the first side 38. As mentioned above, in some embodiments the hypotube 28 may be provided to the interior surface of the lumen 20 of the catheter shaft 12 rather than an inner lumen 26 formed in the wall of the catheter shaft 12.

The catheter shaft 12 can be constructed to have an extended portion 42 of the wall of the catheter shaft 12 that extends distally beyond the opening 24. As shown, the extended portion 42 of the wall of the catheter shaft 12 extends more distal on the first side 38 of the catheter shaft. The portion of the wall of the catheter shaft 12 that does not include the extended portion 42 may be tapered to a thinner thickness than an opposing portion of the wall of the catheter shaft 12. The extended portion 42 of the catheter shaft 12 can span one-half of a circumference of the catheter shaft 12, or may span more or less than one-half of the circumference of the catheter shaft 12.

In these embodiments, the inner lumen 26 terminates in the extended portion 42 of the catheter shaft 12 without opening to the distal end 16 of the catheter shaft 12. However, an aperture 30 is formed on the inner surface of the catheter shaft 12, such that the inner lumen 26 is open to the inner surface of the catheter shaft 12 by way of the aperture 30. The deflection member 22 in this configuration can be coupled to the inner surface of the catheter shaft 12 and can be made to span the aperture 30 such that the deflection member 22 is in fluid communication with a hypotube 28 provided to the inner lumen 26. The deflection member 22 can have a generally hemi-spherical shape that provides a deflection member 22 that is "side-firing" in the sense that the deflection member 22 expands into the lumen 20 of the catheter shaft 12 towards the longitudinal axis 18 of the catheter 10 when expanding from the first volume to the second volume.

The deflection member 22 is coupled to the aperture 30, such that the aperture 30 provides fluid communication between the deflection member and the hypotube 28 positioned in the inner lumen 26 to provide a fluid to the deflection member 22. The fluid provided to the deflection member 22 can facilitate expansion of the deflection member 22 to the second volume as described above. The deflection member 22 at the second volume partially extends into the distal opening 24 of the catheter 10 to provide a surface that will deflect a guidewire or other interventional device extending through the lumen 20 of the catheter shaft 12 by a projection angle, Θ . At the first volume, the deflection member 22 can have a substantially flat shape. As fluid is removed from the deflection member 22 its volume will decrease again from the second volume to the first volume. It should be appreciated that the deflection member 22 can be partially expanded, thereby expanding the deflection member 22 to an intermediate volume.

One example of the embodiment of the catheter 10 as described above can be sized at 4.5 Fr (1.5 mm]. In such embodiments, the catheter 10 can be a referred to as a microcatheter. In some implementations, the catheter 10 may be sized for use in coronary arteries, and in some other implementations the catheter 10 may be sized for use in peripheral arteries.

In another embodiment, the catheter shaft 12 can be constructed to be angled at its distal end 16, as shown in FIG. 7, such that the catheter shaft 12 extends distally farther on one side 41 than on the other side 43 forming a sloped outer surface to the distal end 16 of the catheter shaft 12. In these instances, the inner lumen 26 may terminate within the wall of the catheter shaft 12. Like the embodiments described with respect to FIGS. 5 and 6, an inward facing aperture 30 is formed in the wall of the catheter shaft 12 to provide fluid communication between the inner lumen 26 and a deflection member 22 coupled to the aperture 30. As described above, a hypotube 28 can be provided in the inner lumen 26, or the lumen 20 of the catheter shaft 12, to facilitate providing a fluid to the deflection member 22 to adjust the volume of the deflection member 22 between the first volume and the second volume. In the configuration shown in FIG. 7, the deflection member 22 is arranged opposite the lower side 43 of the catheter shaft 12 at its distal end 16.

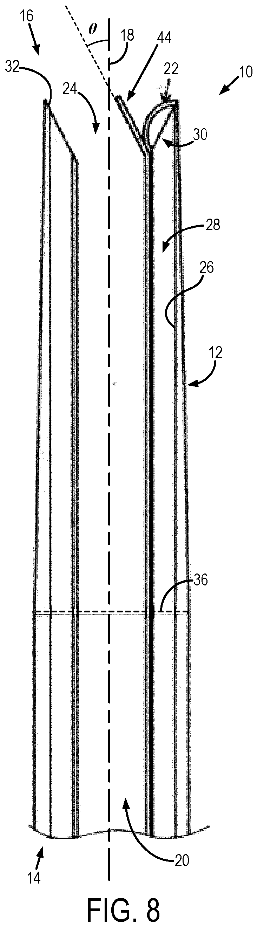

In another embodiment of the catheter 10, a positioning member 44 can be coupled to the catheter shaft 12 at the distal end 16 of the catheter 10, as shown in FIG. 8. The positioning member 44 can be coupled adjacent a deflection member 22, such that expanding the deflection member 22 from a first volume to a second volume will deflect or otherwise articulate the positioning member 44 from a first position to a second position. As an example, in the second position the positioning member 44 can provide a surface for deflecting a guidewire or other interventional device by a deflection angle.

As one example of the embodiments described above, the catheter 10 can be sized at 4.5Fr (1.5 mm]. In some implementations, the catheter 10 may be sized for use in coronary arteries, and in some other implementations the catheter 10 may be sized for use in peripheral arteries.

In operation, the catheter 10 should be properly oriented within the subintimal space, so as to ensure that the guidewire, or other interventional device, extending through the lumen 20 of the catheter 10 will be deflected back into the true lumen of the blood vessel. To this end, the catheter 10 can be constructed to include a radiopaque orientation marker that uniquely indicates the orientation of the catheter shaft 12 within the subintimal space. The catheter 10 can also include a realignment assembly that can be used to reorient the catheter 10 while it resides in the subintimal space, such that the deflection of the guidewire, or other interventional device, will be made into the true lumen of the blood vessel.

Referring now to FIGS. 9 and 10, one example of a radiopaque orientation marker 60 that is disposed on the catheter shaft 12 of the catheter 10 is shown. The radiopaque orientation marker 60 can be positioned on the catheter shaft 12 proximal to a taper line 36, such as those described above. The radiopaque orientation marker 60 is composed of a radiopaque material and generally has an asymmetrical shape around the outer surface of the catheter shaft 12. The asymmetrical shape provides an indication of the orientation of the catheter 10 based on the shape displayed to a user. The catheter 10 can be rotated as will be discussed below, and the rotation of the catheter 10 changes a view of the radiopaque marker 60. As shown in FIGS. 9 and 10, the radiopaque orientation marker 60 may be shaped such that when oriented in a first orientation and viewed along a particular line-of-sight the radiopaque orientation marker 60 will be displayed as a "C- shaped" object in an x-ray image. When the radiopaque orientation marker 60 is rotated to a second orientation and viewed along the same line-of-sight, the radiopaque orientation marker 60 will be displayed as a "Z-shaped" object in an x-ray image. Thus, based on the unique shape of the radiopaque orientation marker 60, a user can visualize the current orientation of the catheter in an x-ray image. It will be appreciated that the radiopaque orientation marker 60 could also be composed of a material that renders it visible in images acquired with other medical imaging modalities, such as magnetic resonance imaging.

To adjust the orientation of the catheter 10, a realignment assembly can be implemented. As shown in FIGS. 13A and 13B, the realignment assembly 62 can generally include a rod 64 that is provided to the catheter shaft 12 of the catheter 10. The rod 64 interfaces with a receiving portion 66 in the catheter shaft 12, at which the rod 64 becomes coupled to the catheter shaft 12. When interfaced with the receiving portion 66 of the catheter shaft 12, rotation of the rod 64 will result in a rotation of the catheter shaft 12, at least at the distal end 16 of the catheter 10, thereby providing a realignment, or reorientation, of the catheter shaft 12.

In the embodiment shown in FIGS. 13A and 13B, the receiving portion 66 includes protrusions 68 disposed on the interior wall of the catheter shaft 12. These protrusions 68 create a decreased diameter of the lumen 20 of the catheter shaft 12. The protrusions 68 may be curved or otherwise shaped to provide a reduced diameter of the lumen 20 of the catheter shaft 12. The protrusions 68 can be semi-deformable. When the rod 64 is provided to the receiving portion 66, the protrusions 68 will contact the distal end of the rod 64, thereby creating an interference fit between the rod 64 and the receiving portion 66. In this arrangement, the rod 64 becomes mechanically coupled to the catheter shaft 12 such that when the rod 64 is rotated it provides a rotation of the catheter shaft 12.

In one embodiment, shown in FIGS. 14A and 14B, the realignment assembly
62 can include a rod 64 that is tapered at its distal end. The receiving portion 66 of the catheter shaft 12 is similarly tapered to receive the tapered rod 64. The tapering of the receiving portion 66 provides a tapered fit with the rod 64 such that the rod 64 becomes mechanically coupled to the catheter shaft 12 when interfaced with the receiving portion 66. As such, when the rod 64 is rotated it provides a rotation of the catheter shaft 12.

In another embodiment shown in FIGS. 15A and 15B, the realignment assembly 62 can include a rod 64 that is shaped at its distal end to mate with the receiving portion 66 of the catheter shaft 12. For example, the rod 64 and receiving portion 66 can collectively define a "lock and key" mechanism. The receiving portion 66 can include an annular stopper 70 coupled to the inner wall of the catheter shaft 12 and having one or more notched recesses 72 that receive similarly shaped protrusions 74 extending distally from the distal end of the rod 64. In some configurations, such as the one shown in FIGS. 15A and 15B, the notched recesses can be mirrored curved recesses that are recessed from the proximal surface of the stopper 70 opposite each other. The keyed end of the rod 64 can have a cylindrical central member 76 and opposing curved protrusions 74 that are shaped to interface with the recesses 72 and the annular stopper 70. When the keyed end of the rod 64 is interfaced with the recesses 72 in the annular stopper 70, the rod 64 becomes mechanically coupled to the catheter shaft 12 such that when the rod 64 is rotated it provides a rotation of the catheter shaft 12.

Referring to FIG. 16, at its proximal end the rod 64 can have a handle 78 that is cylindrical in shape with a tapered distal end that tapers radially inward to meet the rod 64. The outer surface of the handle 78 can feature a plurality of ribs 80 having a raised profile and extending along a length of the outer surface of the handle 78. The rod 64 can be generally cylindrical in shape and can extend to any appropriate length to its distal end. Referring to FIG. 17, as another example, the handle 78 of the rod 64 can be generally cone-shaped being tapered radially inward to meet the rod 64 at a distal end of the handle 78. Referring to FIG. 18, as another example, handle 78 of the rod 64 can have a proximal body 82 that is generally spherical in shape and a distal body 84 that is generally cylindrical in shape and interfaces with the rod 64.

Having generally described the features of the various embodiments of the catheter 10, a discussion of its general mode of operation is provided. By way of example, the operation of the various embodiments of the catheter 10 will be described with respect to treatment of chronic total occlusions in a patient. The catheter 10 can be configured as a re-entry component for re-entering a true lumen of a patient once the catheter 10 has been oriented at a desired orientation in the subintimal space. In percutaneous revascularization therapy, it is desirable to have the catheter 10 positioned after the chronic total occlusionfs] prior to re-entry in order to facilitate the procedure. As noted above, it should be appreciated by those skilled in the art that the catheter 10 can be employed for other procedures.

The catheter 10 can be positioned in the subintimal space of a patient near an occlusion designated for treatment. The deflection member 22 receives fluid from a hypotube 28 disposed within the inner lumen 26 or provided to the interior surface of the lumen 20 of the catheter shaft 12, and the fluid causes the deflection member 22 to expand from the first volume to the second volume. The fluid supplied to the deflection member 22 can be controlled by a user at a proximal end 14 of the catheter 10. As one example, the fluid can be supplied via a syringe, similar to those used in balloon catheters.

When the deflection member 22 is expanded to the second volume, it partially extends into the distal opening 24 of the catheter 10 to provide a surface that will deflect a guidewire or other interventional instrument by a projection angle, Θ . It will be appreciated that during a percutaneous revascularization procedure multiple different guidewires can be interchangeably used with the catheter 10. For instance, the guidewire can be changed for a stiffer or slippery guidewire to penetrate through the subintimal tissue to facilitate reentry into the vessel lumen. The deflection surface can be positioned on an interior side of the deflection member 22 such that when the guidewire 34 extends through the distal opening 24 of the lumen 20 of the catheter 10, , the guidewire 34 will make contact with and be deflected by the surface of the deflection member. Contact between the surface of the deflection member 22 and the guidewire 34 deflects the guidewire 34 through the distal opening 24 along the projection angle, Θ . The guidewire 34 may also contact the angled surface 32 that provides proximal support to the guidewire 34 when deflected through the distal opening 24. The guidewire 34 can extend distally from the distal end 16 of the catheter device along the projection angle, Θ . The projection angle, θ , can be determined by a user pre-operatively or during operation in order to facilitate re-entry into the true lumen beyond the occlusion and can be selectively controlled by adjusting the volume of the deflection member 22 as needed. The catheter 10 can then be advanced over the guidewire 34 into the true lumen beyond the occlusion.

During revascularization therapy using the catheter devices described in the present disclosure, it is generally desirable for the user to understand an orientation of the catheter device in order to determine the proper projection angle and to ensure the catheter device is oriented properly such that the projection angle is positioned for re-entry into the true lumen. Accordingly, the radiopaque orientation marker 60 described above and shown in FIGS. 9-12 can be used to assist in alignment of the catheter 10. The asymmetrical shape of the radiopaque orientation marker 60 provides an indication of the orientation of the catheter 10 based on the appearance of the shape of the radiopaque orientation marker 60 displayed in an x-ray or other medical image of the catheter 10.

Rotation of the catheter 10 to a desired orientation can be achieved using the realignment assembly described above. By causing rotation of the rod 64 while it is interfaced with the receiving portion 66 of the catheter shaft, the catheter 10 can be rotated between different orientations. The rod 64 can be stiff and allow for increased translation of rotation applied by a user at the handle 78 located at the proximal end of the rod 64. The rod 64 can also provide support along the length of the catheter shaft 12 to rotate the catheter shaft 12 without kinking. Once the desired orientation is achieved, the rod 64 can be removed from the lumen 20 of the catheter shaft 12 and then be replaced with a guidewire for re-entry into the true lumen as discussed above.

Thus, as one non-limiting example of its use, the catheter 10 will be oriented in the proper direction beyond the occlusion using the radiopaque orientation marker 60 and the orientation rod 64. A guidewire will be advanced to the tip of the catheter 10, beside the uninflated deflection member 22. The deflection member 22 will then be expanded, angling the guidewire into the vessel lumen. The guidewire will then be advanced into the true lumen beyond the occlusion. The catheter 10 will then be advanced over the guidewire and into the true lumen to secure the position. After the guidewire has been advanced into the distal lumen, the catheter 10 in the subintimal space may be exchanged for a balloon catheter or different microcatheter while maintaining the wire position in the distal lumen.

As described, the catheter 10 can be used in CTO interventions, specifically directing a guidewire from the subintimal space towards the true lumen. The catheter 10 can also be used for directing a guidewire down a difficult to access side branch (in narrowed but not occluded arteries], for example.

Referring now to FIGS. 19A-19G, an example method for using the catheter 10 described in the present disclosure for a subintimal re-entry procedure is shown. A section of an artery with a chronic total occlusion is shown in FIG 19A. A surgeon positions a guidewire to enter the subintimal space proximal to the occlusive plaque, as shown in FIG. 19B. The microcatheter is then tracked over the guidewire into the subintimal space, as shown in FIG. 19C. The microcatheter is in the correct orientation for re-entry to the true lumen, as indicated by the "C" shaped marker facing towards the true lumen. If, however, the microcatheter is tracked over the guidewire into the subintimal space and catheter is not in the correct orientation for re-entry to the true lumen, as shown in FIG. 19D and indicated by the backwards "Z" shaped marker facing towards the true lumen, the orientation rod is inserted into the microcatheter to re-orient the microcatheter until the "C" shaped marker faces towards the true lumen. The guidewire is then pulled back inside the microcatheter until just the distal tip of the guidewire protrudes out of the opening, as shown in FIG. 19E. The expandable membrane of the deflection member is then inflated to angulate the distal tip of the guidewire, which can then be advanced to re-enter the true lumen, as shown in FIG. 19F. The catheter is then advanced over the guidewire into the true lumen, as shown in FIG, 19G. Alternatively, the guidewire is advanced into the distal lumen, and the microcatheter is withdrawn and can be replaced with a different catheter

Referring now to FIGS. 20A-20G, an example method for using the catheter
10 described in the present disclosure for accessing a difficult to reach side branch is shown. In this example, the difficult to reach side branch is in an atherosclerotic right coronary artery, as shown in FIG. 2 OA. A surgeon positions a guidewire to enter the right coronary artery, but cannot access the side branch, as shown in FIG. 20B. The microcatheter is tracked over the guidewire, as shown in FIG. 20C. In this example, the microcatheter is in the correct orientation for angulating the guidewire into the side branch, as indicated by the "C" shaped marker facing towards the side branch. If, however, the microcatheter is tracked over the guidewire and is not in the correct orientation for angulating the guidewire into the side branch, as shown in FIG. 20D and indicated by the backwards "Z" shaped marker facing towards the side branch, then the orientation rod is inserted into the microcatheter to re-orient the microcatheter until the "C" shaped marker faces towards the side branch. The guidewire is then pulled back inside the microcatheter until just the distal tip of the guidewire protrudes out of the opening, as shown in FIG. 20E. The expandable membrane of the deflection member is inflated to angulate the distal tip of the guidewire, which can then be advanced to enter the side branch, as shown in FIG. 2 OF. When the guidewire successfully accessed the side branch, the microcatheter can either be advanced over the guidewire, or replaced with a different catheter, as shown in FIG. 20G.

One advantage of the catheter 10 described in the present disclosure is that the configuration can be constructed to maintain the outer dimensions of a microcatheter, and will have a lower risk of causing harm to the artery or the subintimal space when used in the coronary arteries. That is, the deflection member 22 and other components of the catheter 10 described in the present disclosure are located within the catheter shaft 12, and the dimensions of these components can thus be sized so as not to exceed the microcatheter outer diameter. The ability of the catheter 10 to direct the guidewire out of the distal tip also allows for the catheter 10 to be advanced across a lesion without requiring the catheter 10 to be replaced.

The catheter 10 described in the present disclosure can enable cardiologists to successfully perform percutaneous coronary interventions for complex chronic total occlusions. The catheter 10 can allow for an alternative method for directing a guidewire from the subintimal space into the true lumen that can reduce procedural costs and have a lower risk of complications as compared to current devices. Another advantage is that because the catheter 10 stays in place throughout the subintimal entry, little to no blood will track through the subintimal plane of tissue, which can otherwise occur when the a device (e.g., the CrossBoss described above] is removed and replaced with another device (e.g., the Stingray described above]. As a result, the subintimal space will be prevented from filling up with blood and compressing the true lumen. In turn, the subintimal entry is made easier because the true lumen beyond the CTO is maintained. If the true lumen is compressed by blood tracking in the subintimal space, the distal lumen may become very difficult to visualize.

The present invention has been described in terms of one or more preferred embodiments, and it should be appreciated that many alternatives, variations, and modifications, aside from those expressly stated, are possible and within the scope of the invention.

## Claims

1. A catheter (10) comprising:
a catheter shaft (12) having a tubular wall that extends from a proximal end (14) to a distal end (16) along a longitudinal axis (18) to define a lumen (20), the tubular wall having formed therein an inner lumen (26) that extends from the proximal end to the distal end of the catheter shaft;
a deflection member (22) coupled to the distal end of the catheter shaft and in fluid communication with the inner lumen such that fluid provided to the inner lumen causes the deflection member to expand from a first volume to a second volume that is larger than the first volume;
wherein when the deflection member is in the second volume it extends from a surface of the tubular wall towards the longitudinal axis of the catheter shaft to provide a surface for deflecting an interventional device extending through the lumen and outward from the distal end of the catheter shaft at a deflection angle; and
wherein the distal end of the tubular wall includes an angled surface (32) that is angled from an outer surface of the tubular wall proximally towards an inner surface of the tubular wall.

2. The catheter of claim 1, wherein the deflection member is in fluid communication with a hypotube (28) positioned within the inner lumen of the catheter shaft.

3. The catheter of claim 1, further comprising:
a flexible guidewire (34) dimensioned to be received through and conforming to a shape of the lumen and having a distal end that extends outwardly through a distal opening; and
wherein the deflection member is configured to angularly displace the distal end of the flexible guidewire at the deflection angle.

4. The catheter of claim 1, wherein the deflection member is coupled to the angled surface and when in the second volume expands away from the angled surface along a direction that is normal to the angled surface.

5. The catheter of claim 4, further comprising a positioning member (44) rotatably coupled to the inner surface of the tubular wall proximal to the angled surface such that when the deflection member is in the first volume the positioning member is in a first position that is parallel with the longitudinal axis and when the deflection member is in the second volume the positioning member is contacted by the deflection member and articulated into a second position that is angled at the deflection angle.

6. The catheter of claim 1, wherein the deflection member is coupled to an inner surface of the tubular wall and when in the second volume expands away from the inner surface of the tubular wall along a direction that is normal to the inner surface.

7. The catheter of claim 1, further comprising a radiopaque orientation marker (60) disposed on an outer surface of the tubular wall and having an asymmetrical shape that indicates an orientation of the catheter to a user.

8. The catheter of claim 1, further comprising a realignment assembly (62) coupled to the tubular wall such that rotation of the realignment assembly about the longitudinal axis results in a rotation of the catheter shaft about the longitudinal axis.

9. The catheter of claim 8, wherein the realignment assembly comprises a receiving portion (66) formed in the tubular wall and a rod (64) that is sized to be received by the receiving portion of the tubular wall and when received by the receiving portion of the tubular wall mechanically couples the rod to the catheter shaft.

10. The catheter of claim 9, wherein the receiving portion includes a tapered surface that extends from a proximal end to a distal end along the longitudinal axis and tapers from a diameter of the lumen at its proximal end to a smaller diameter at its distal end, and wherein a distal end of the rod is tapered to be received by the receiving portion.

11. The catheter of claim 9, wherein the receiving portion comprises one or more deformable protrusions (68) extending outward from an inner surface of the tubular wall in order to provide an interference fit with the rod or wherein the receiving portion comprises an annular stopper (70) formed in the tubular wall and having formed therein one or more notched recesses, recesses (72), and wherein the rod has formed at its distal end one or more protrusions (74) that interface with the one or more notched recesses in the annular stopper.

12. The catheter of claim 2, further comprising:
a first aperture formed in the hypotube, wherein the deflection member is coupled to the hypotube at the first aperture; and
a second aperture formed in the inner lumen that is aligned with the first aperture, such that when the deflection member is expanded from the first volume to the second volume the deflection member extends through the second aperture towards the longitudinal axis of the catheter shaft.

13. The catheter of claim 12, wherein the first aperture is formed at the distal end of the hypotube and the second aperture is formed at the distal end of the catheter shaft or wherein the first aperture is formed on a circumference of the hypotube and directed toward the longitudinal axis of the catheter shaft, and the second aperture is formed on an inner surface of the catheter shaft and directed towards the longitudinal axis of the catheter shaft.

14. The catheter of claim 12, wherein the deflection member comprises an extruded sleeve (50) that is arranged over the first aperture, wherein the extruded sleeve is composed of an expandable material or wherein the deflection member comprises a patch (52) formed over the first aperture, wherein the patch is composed of an expandable material or wherein the deflection member is constructed by dipping the hypotube in an expandable material such that the expandable material spans the first aperture.

15. The catheter of claim 14, wherein the expandable material is selected from the group consisting essentially of silicon, silicone polypropylene, polyurethane, or polyether block amide.

## Patentansprüche

1. Katheter (10) umfassend:
einen Katheterschaft (12) mit einer tubularen Wand, die sich von einem proximalen Ende (14) zu einem distalen Ende (16) entlang einer Längsachse (18) erstreckt, um ein Lumen (20) zu definieren, wobei die tubulare Wand in ihr ein inneres Lumen (26) ausgebildet hat, das sich von dem proximalen Ende zu dem distalen Ende des Katheterschafts erstreckt;
ein Ablenkelement (22), das mit dem distalen Ende des Katheterschafts gekoppelt ist und derart in Fluidverbindung mit dem inneren Lumen steht, dass das Fluid, das zu dem inneren Lumen bereitgestellt wird, das Ablenkelement veranlasst, sich von einem ersten Volumen auf ein zweites Volumen zu erstrecken, das größer als das erste Volumen ist;
wobei, wenn sich das Ablenkelement in dem zweiten Volumen befindet, es sich von einer Fläche der tubularen Wand in Richtung der Längsachse des Katheterschafts erstreckt, um eine Fläche zum Ablenken einer Eingriffsvorrichtung bereitzustellen, die sich durch das Lumen und nach außen von dem distalen Ende des Katheterschafts in einem Ablenkwinkel erstreckt; und
wobei das distale Ende der tubularen Wand eine winklige Fläche (32) aufweist, die von einer Außenfläche der tubularen Wand proximal zu einer Innenfläche der tubularen Wand angewinkelt ist.

2. Katheter nach Anspruch 1, wobei das Ablenkelement in Fluidverbindung mit einem Hypotubus (28) steht, der innerhalb des inneren Lumens des Katheterschafts positioniert ist.

3. Katheter nach Anspruch 1, ferner umfassend:
einen flexiblen Führungsdraht (34), der so dimensioniert ist, dass er durch und entsprechend einem Verlauf des Lumens aufgenommen wird und ein distales Ende aufweist, das sich nach außen durch eine distale Öffnung erstreckt; und
wobei das Ablenkelement so konfiguriert ist, dass es das distale Ende des flexiblen Führungsdrahts unter dem Ablenkwinkel winklig verschiebt.

4. Katheter nach Anspruch 1 wobei, dass das Ablenkelement mit der angewinkelten Fläche gekoppelt ist und sich im zweiten Volumen ausgehend von der angewinkelten Fläche in einer Richtung senkrecht zur angewinkelten Fläche erstreckt.

5. Katheter nach Anspruch 4, ferner umfassend ein Positionierungselement (44), das drehbar mit der Innenfläche der tubularen Wand proximal zu der angewinkelten Fläche derart gekoppelt ist, dass wenn das Ablenkelement in dem ersten Volumen ist, das Positionierungselement in einer ersten Position ist, die parallel zur Längsachse ist, und wenn das Ablenkelement in dem zweiten Volumen ist, das Positionierungselement von dem Ablenkelement kontaktiert und in eine zweite Position angewinkelt verbunden ist, die in dem Ablenkwinkel angewinkelt ist.

6. Katheter nach Anspruch 1, wobei das Ablenkelement mit einer Innenfläche der tubularen Wand gekoppelt ist und sich im zweiten Volumen von der Innenfläche der tubularen Wand entlang einer zur Innenfläche senkrechten Richtung ausdehnt.

7. Katheter nach Anspruch 1, ferner umfassend eine röntgenopake Orientierungsmarkierung (60), die auf einer Außenfläche der tubularen Wand angeordnet ist und einen asymmetrischen Verlauf aufweist, die eine Orientierung des Katheters für einen Benutzer anzeigt.

8. Katheter nach Anspruch 1, ferner umfassend eine Neuausrichtungsbaugruppe (62), die mit der tubularen Wand derart gekoppelt ist, dass eine Drehung der Neuausrichtungsbaugruppe um die Längsachse zu einer Drehung des Katheterschafts um die Längsachse führt.

9. Katheter nach Anspruch 8, wobei die Neuausrichtungsbaugruppe einen Aufnahmeabschnitt (66), der in der tubularen Wand ausgebildet ist, und einen Stab (64) umfasst, der so bemessen ist, dass er von dem Aufnahmeabschnitt der tubularen Wand aufgenommen wird, und wenn er von dem Aufnahmeabschnitt der tubularen Wand aufgenommen wird, verbindet er den Stab mechanisch mit dem Katheterschaft.

10. Katheter nach Anspruch 9, wobei der Aufnahmeabschnitt eine verjüngte Fläche aufweist, die sich von einem proximalen Ende zu einem distalen Ende entlang der Längsachse erstreckt und sich von einem Durchmesser des Lumens an seinem proximalen Ende zu einem kleineren Durchmesser an seinem distalen Ende verjüngt, und wobei ein distales Ende des Stabs verjüngt ist, um von dem Aufnahmeabschnitt aufgenommen zu werden.

11. Katheter nach Anspruch 9, wobei der Aufnahmeabschnitt einen oder mehrere verformbare Vorsprünge (68) umfasst, die sich von einer Innenfläche der tubularen Wand nach außen erstrecken, um einer Presspassung mit dem Stab bereitzustellen, oder wobei der Aufnahmeabschnitt einen ringförmigen Stopfen (70) umfasst, der in der tubularen Wand ausgebildet ist und eine oder mehrere Kerbaussparungen, Aussparungen (72) aufweist, und wobei der Stab an seinem distalen Ende einen oder mehrere Vorsprünge (74) ausgebildet hat, die mit der einen oder den mehreren Kerbaussparung in dem ringförmigen Stopfen zusammenwirken.

12. Der Katheder nach Anspruch 2, ferner umfassend:
eine erste Öffnung, die in dem Hypotubus ausgebildet ist, wobei das Ablenkelement
mit dem Hypotubus an der ersten Öffnung gekoppelt ist; und
eine zweite Öffnung, die in dem inneren Lumen ausgebildet ist, das mit der ersten Öffnung derart ausgerichtet ist, dass sich das Ablenkelement beim Aufweiten des Ablenkelements von dem ersten Volumen auf das zweite Volumen durch die zweite Öffnung in Richtung der Längsachse des Katheterschafts erstreckt.

13. Katheter nach Anspruch 12, wobei die erste Öffnung am distalen Ende des Hypotubus ausgebildet ist und die zweite Öffnung am distalen Ende des Katheterschafts ausgebildet ist oder wobei die erste Öffnung an einem Umfang des Hypotubus ausgebildet ist und zu der Längsachse des Katheterschafts gerichtet ist, und die zweite Öffnung an einer Innenfläche des Katheterschafts ausgebildet ist und zu der Längsachse des Katheterschafts gerichtet ist.

14. Katheter nach Anspruch 12, wobei das Ablenkelement eine extrudierte Hülse (50) umfasst, die über der ersten Öffnung angeordnet ist, wobei die extrudierte Hülse aus einem expandierbaren Material besteht oder wobei das Ablenkelement ein über der ersten Öffnung gebildetes Patch (52) umfasst, wobei das Patch aus einem expandierbaren Material besteht oder wobei das Ablenkelement durch Eintauchen des Hypotubus in ein expandierbares Material derart aufgebaut ist, dass das expandierbare Material die erste Öffnung überspannt.

15. Katheter nach Anspruch 14, wobei das expandierbare Material ausgewählt ist aus der Gruppe, die im Wesentlichen aus Silicium, Silikon, Polypropylen, Polyurethan oder Polyetherblockamid besteht.

## Revendications

1. Cathéter (10) comprenant :
une tige de cathéter (12) ayant une paroi tubulaire qui se prolonge d'une extrémité proximale (14) à une extrémité distale (16) le long d'un axe longitudinal (18) pour définir une lumière (20), une lumière interne (26) qui se prolonge de l'extrémité proximale à l'extrémité distale de la tige de cathéter étant formée à l'intérieur de la paroi tubulaire ;
un élément de déviation (22) couplé à l'extrémité distale de la tige de cathéter et en communication fluidique avec la lumière interne de sorte que le fluide fourni à la lumière interne amène l'expansion de l'élément de déviation d'un premier volume à un second volume qui est plus grand que le premier volume ;
dans lequel, lorsque l'élément de déviation est dans le second volume, il se prolonge d'une surface de la paroi tubulaire vers l'axe longitudinal de la tige de cathéter pour fournir une surface permettant de dévier un dispositif d'intervention se prolongeant à travers la lumière et vers l'extérieur de l'extrémité distale de la tige de cathéter selon un angle de déviation ; et
dans lequel l'extrémité distale de la paroi tubulaire comporte une surface inclinée (32) qui est inclinée à partir d'une surface extérieure de la paroi tubulaire de manière proximale vers une surface intérieure de la paroi tubulaire.

2. Cathéter selon la revendication 1, dans lequel l'élément de déviation est en communication fluidique avec un hypotube (28) positionné à l'intérieur de la lumière interne de la tige de cathéter.

3. Cathéter selon la revendication 1, comprenant également :
un fil de guidage flexible (34) dimensionné pour être reçu à travers et se conformer à une forme de la lumière et ayant une extrémité distale qui se prolonge vers l'extérieur à travers une ouverture distale ; et
dans lequel l'élément de déviation est configuré pour déplacer angulairement l'extrémité distale du fil de guidage flexible selon l'angle de déviation.

4. Cathéter selon la revendication 1, dans lequel l'élément de déviation est couplé à la surface inclinée et, lorsqu'il est dans le second volume, se dilate en s'éloignant de la surface inclinée le long d'une direction qui est perpendiculaire à la surface inclinée.

5. Cathéter selon la revendication 4, comprenant également un élément de positionnement (44) couplé de manière rotative à la surface intérieure de la paroi tubulaire à proximité de la surface inclinée de sorte que lorsque l'élément de déviation est dans le premier volume, l'élément de positionnement est dans une première position qui est parallèle à l'axe longitudinal et lorsque l'élément de déviation est dans le second volume, l'élément de positionnement est en contact avec l'élément de déviation et articulé dans une seconde position qui est inclinée selon l'angle de déviation.

6. Cathéter selon la revendication 1, dans lequel l'élément de déviation est couplé à une surface intérieure de la paroi tubulaire et, lorsqu'il est dans le second volume, se dilate en s'éloignant de la surface intérieure de la paroi tubulaire le long d'une direction qui est perpendiculaire à la surface intérieure.

7. Cathéter selon la revendication 1, comprenant également un marqueur d'orientation radio-opaque (60) disposé sur une surface extérieure de la paroi tubulaire et ayant une forme asymétrique qui indique une orientation du cathéter vers un utilisateur.

8. Cathéter selon la revendication 1, comprenant également un ensemble de réalignement (62) couplé à la paroi tubulaire de sorte que la rotation de l'ensemble de réalignement autour de l'axe longitudinal entraîne une rotation de la tige de cathéter autour de l'axe longitudinal.

9. Cathéter selon la revendication 8, dans lequel l'ensemble de réalignement comprend une partie de réception (66) formée dans la paroi tubulaire et une tige (64) qui est dimensionnée pour être reçue par la partie de réception de la paroi tubulaire et qui, lorsqu'elle est reçue par la partie de réception de la paroi tubulaire, couple mécaniquement la tige à la tige de cathéter.

10. Cathéter selon la revendication 9, dans lequel la partie de réception comporte une surface conique qui se prolonge d'une extrémité proximale à une extrémité distale le long de l'axe longitudinal et se rétrécit d'un diamètre de la lumière au niveau de son extrémité proximale à un diamètre plus petit au niveau de son extrémité distale, et dans lequel une extrémité distale de la tige est conique pour être reçue par la partie de réception.

11. Cathéter selon la revendication 9, dans lequel la partie de réception comprend une ou plusieurs saillies déformables (68) se prolongeant vers l'extérieur à partir d'une surface intérieure de la paroi tubulaire afin de fournir un ajustement serré avec la tige ou dans lequel la partie de réception comprend un bouchon annulaire (70) formé dans la paroi tubulaire et un ou plusieurs évidements crantés, évidements (72) étant formés à l'intérieur et dans lequel une ou plusieurs saillies (74) qui s'interfacent avec les un ou plusieurs évidements crantés sont formées au niveau de l'extrémité distale de la tige dans le bouchon annulaire.

12. Cathéter selon la revendication 2, comprenant également :
une première ouverture formée dans l'hypotube, dans lequel l'élément de déviation est
couplé à l'hypotube au niveau de la première ouverture ; et
une seconde ouverture formée dans la lumière interne qui est alignée avec la première ouverture, de sorte que lorsque l'élément de déviation est dilaté du premier volume au second volume, l'élément de déviation se prolonge à travers la seconde ouverture vers l'axe longitudinal de la tige de cathéter.

13. Cathéter selon la revendication 12, dans lequel la première ouverture est formée au niveau de l'extrémité distale de l'hypotube et la seconde ouverture est formée au niveau de l'extrémité distale de la tige de cathéter ou dans lequel la première ouverture est formée sur une circonférence de l'hypotube et dirigée vers l'axe longitudinal de la tige de cathéter, et la seconde ouverture est formée sur une surface intérieure de la tige de cathéter et dirigée vers l'axe longitudinal de la tige de cathéter.

14. Cathéter selon la revendication 12, dans lequel l'élément de déviation comprend un manchon extrudé (50) qui est disposé sur la première ouverture, dans lequel le manchon extrudé est composé d'un matériau extensible ou dans lequel l'élément de déviation comprend un patch (52) formé sur la première ouverture, dans lequel le patch est composé d'un matériau extensible ou dans lequel l'élément de déviation est construit en trempant l'hypotube dans un matériau extensible de sorte que le matériau extensible couvre la première ouverture.

15. Cathéter selon la revendication 14, dans lequel le matériau extensible est choisi dans le groupe constitué essentiellement de silicium, de polypropylène de silicone, de polyuréthane ou de polyéther bloc amide.
